(19) Europäisches Patentamt
European Patent Office
Office européen des brevets

(11) Publication number: 0 031 218
B1

(12) **EUROPEAN PATENT SPECIFICATION**

(45) Date of publication of patent specification: 11.05.83

(51) Int. Cl.³: C 07 D 213/73 //A01N47/36, C07D213/75

(21) Application number: 80304466.8

(22) Date of filing: 11.12.80

(54) **A 2-amino-trifluoromethyl-halogenopyridine compound and a process for producing the same.**

(30) Priority: 24.12.79 JP 167896/79

(43) Date of publication of application:
01.07.81 Bulletin 81/26

(45) Publication of the grant of the patent:
11.05.83 Bulletin 83/19

(84) Designated Contracting States:
BE CH DE FR GB IT LI NL

(56) References cited:
EP - A - 0 001 473
EP - A - 0 003 278
GB - A - 1 417 349
US - A - 3 609 158

The file contains technical information submitted after the application was filed and not included in this specification

(73) Proprietor: ISHIHARA SANGYO KAISHA LTD.
No. 3-11, Edobori 1-chome
Nishi-ku Osaka-shi Osaka 550 (JP)

(72) Inventor: Isao, Yokomichi
No. 321-31 Fuke-cho
Moriyama-shi Shiga (JP)
Inventor: Takahiro, Haga
No. 84-7 Hirai-cho
Kusatsu-shi Shiga (JP)
Inventor: Kuniaki, Nagatani
No. 3-56-21 Yagura-cho 1-chome
Kusatsu-shi Shiga (JP)
Inventor: Toshio, Nakajima
No. 15-22, Nishi Shibukawa 1-Chome
Kusatsu-shi Shiga (JP)

(74) Representative: Roberts, Peter William et al,
Marks & Clerk Alpha Tower Suffolk Street
Queensway
Birmingham B1 1TT (GB)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England

A 2-amino-trifluoromethyl-halogenopyridine compound and a process for producing the same

This invention relates to a 2-amino-trifluoromethyl-halogenopyridine compound and a process for producing the same.

A number of pyridine derivatives have hitherto been known to be useful intermediates for the production of organic compounds. For example, 2-amino-5-trifluoromethylpyridine as a starting material for the production of imidazopyridines and a process for the production thereof from 5-carboxy-2-hydroxypyridine are described in US - A - 3 681 369.

2-chloro-3-amino-5-trifluoromethylpyridine and 2-chloro-4-trifluoromethyl-6-aminopyridine are disclosed in EP - A - 0 003 278 and US - A - 3 609 158, respectively, as intermediates useful for the preparation of pharmaceutical compositions and herbicidal compositions, respectively.

The 2-amino-trifluoromethyl-halogenopyridine compounds of this invention are useful as intermediates in the synthesis of particularly effective agricultural chemicals and medicines.

The invention resides in one aspect in a 2-amino-trifluoromethyl-halogenopyridine compound characterised by the formula (I):

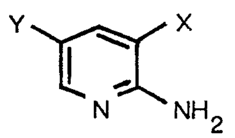

(I)

wherein either X or Y is a trifluoromethyl group and the other is a chlorine atom or a bromine atom.

In a further aspect, the invention resides in a process for the production of the 2-amino-trifluoromethyl-halogenopyridine compound of the formula (I), characterised by reacting a 2-halogeno-trifluoromethyl-halogeno-pyridine represented by the formula (II):

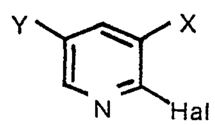

(II)

wherein X and Y are the same as defined hereinbefore, and Hal is a halogen atom, with ammonia at a temperature of 50 to 180°C.

In formula (II) above, Hal may be fluorine, chlorine, bromine or iodine.

In yet a further aspect, the invention resides in a process for the production of a 2-amino-trifluoromethyl-halogenopyridine compound represented by the formula (I):

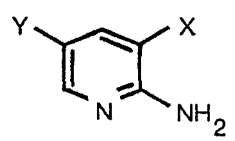

(I)

wherein either X or Y is a trifluoromethyl group and the other is a chlorine atom or a bromine atom, characterised by reacting a 2-halogeno-trifluoromethylpyridine compound represented by the formula (III):

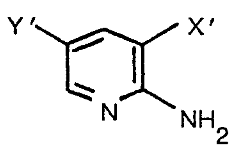

(III)

wherein either X′ or Y′ is a trifluoromethyl group and the other is a hydrogen atom, with a chlorinating or brominating agent.

Examples of 2-amino-trifluoromethyl-halogenopyridines of this invention include 2-amino-3-chloro-5-trifluoromethylpyridine (m.p. 90—92°C), 2-amino-3-bromo-5-trifluoromethylpyridine (m.p. 99—100°C), 2-amino-3-trifluoromethyl-5-bromopyridine (m.p. 84—86°C) 2-amino-3-trifluoromethyl-5-chloropyridine (m.p. 85—87°C).

The 2-amino-trifluoromethyl-halogenopyridine of this invention can be usually produced by the following Method (1) or (2).

*Method (1):* Amination of 2-Halogeno Compound

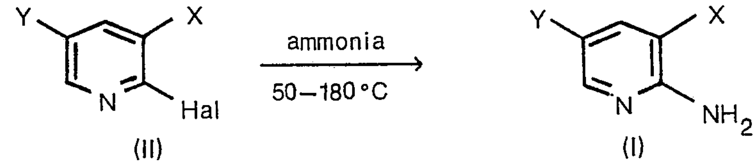


In the formulae (I) and (II), X, Y and Hal are the same as defined hereinbefore.

A 2-halogeno-trifluoromethyl-halogenopyridine compound of the formula (II) and aqueous ammonia which has previously been prepared by dissolving ammonia in water are placed in a closed vessel, e.g., an autoclave, or liquid ammonia is introduced into the 2-halogeno-trifluoromethyl-halogenopyridine compound, followed by the reaction at a temperature of 50° to 180°C, preferably 100° to 150°C. When the reaction temperature exceeds the above specified upper limit, the desired product tends to decompose, whereas when it is below the lower limit, the reaction does not proceed sufficiently.

Where aqueous ammonia is used as the ammonia, one having a concentration of 20% or more, preferably 28 to 40% is usually used. The pressure during the reaction reaches about 2 to 30 atms. ($2.029 \times 10^{-5}$ to $3.042 \times 10^{-6}$ Pa) due to the temperature increase inside the closed vessel.

The amount of ammonia used is 1 mol or more, preferably 3 to 10 mols, per mol of the 2-halogeno-trifluoromethyl-halogenopyridine. The time of reaction is 3 hours or longer, preferably 3 to 12 hours.

The reaction product thus obtained is allowed to cool and usually extracted with a solvent, such as methylene chloride, benzene, or diethyl ether. The solvent is then evaporated off, and if necessary, usual distillation is carried out to obtain the desired product, 2-amino-trifluoromethyl-halogenopyridine. The desired product in the reaction product is obtained as an oily substance or crystals, and thus, impurities can be removed by the above extraction.

Further, in the event that aqueous ammonia is used as the ammonia, the desired product may be contained in the aqueous phase of the reaction product, but the desired product can sufficiently be recovered by the above extraction.

*Method (2):* Halogenation of 2-Amino Compound

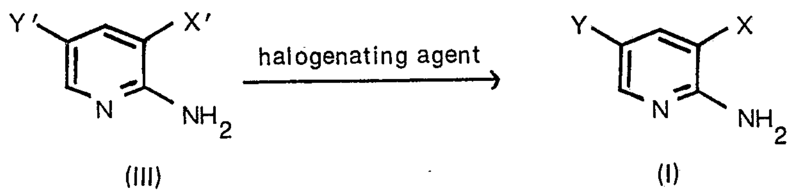


In the formula (III), X′ and Y′ are the same as defined hereinbefore.

2-Amino-trifluoromethylpyridine which can be prepared by the Method (1) above or equivalent method thereto is reacted with a halogenating agent to form a desired 2-amino-trifluoromethyl-halogeno-pyridine. This reaction is usually carried out by introducing the halogenating agent (usually, chlorine gas or bromine) into a solution prepared by dissolving the above starting pyridine in acetic acid, hydrochloric acid, sulfuric acid or hydrobromic acid, and if necessary, by further adding thereto another inert solvent.

For example, in the case of chlorination, the starting pyridine is dissolved in a suitable concentration of hydrochloric acid in an amount of 4 to 10 mols per mol of the starting pyridine, and chlorine gas is introduced into the solution thus prepared while maintaining the temperature of the system at 0° to 100°C, preferably 0° to 50°C. The amount of the chlorine used is usually 1.1 to 5 mols, preferably 1.5 to 3 mols, per mol of the starting pyridine.

In the case of bromination, the starting pyridine is dissolved in an aqueous solution of acetic acid or hydrobromic acid in an amount of 5 to 20 mols per mol of the starting pyridine, and bromine is dropwise added thereto while maintaining the temperature of the system at 10° to 60°C, preferably 35° to 45°C. The amount of the bromine used is usually 1 to 2 mols, preferably 1 to 1.2 mols, per mol of the starting pyridine.

The reaction is usually completed in 0.5 to 10 hours although it may vary depending upon the reaction conditions.

The substitution reaction of the hydrogen atom by the halogen atom occurs at the β-position of the pyridine nucleus at which no trifluoromethyl group exists.

The present invention will be described in detail by reference to the following Examples which, however, do not limit the present invention in any way.

Example 1

Preparation of 2-Amino-3-chloro-5-trifluoromethylpyridine

6.5 g of 2,3-dichloro-5-trifluoromethylpyridine and 20 ml of 28% aqueous ammonia were placed in a 50 ml autoclave, and the mixture was reacted for 24 hours at 100°C, and further for 5 hours at 125°C (inner pressure: about 2 atms.). After completion of the reaction, the reaction product was allowed to cool to obtain crystals. The thus obtained crystals were then washed with water and dried to obtain 1.5 g of 2-amino-3-chloro-5-trifluoromethylpyridine having a melting point of 90 to 92°C.

Example 2

Preparation of 2-Amino-3-bromo-5-trifluoromethylpyridine

In a 100 ml four necked flask equipped with a thermometer a stirrer and a reflux condensor were placed 3g of 2-amino-5-trifluoromethylpyridine and 30 ml of acetic acid to provide a homogeneous solution. 4.4 g of bromine was dropwise added thereto while cooling the flask with ice water at 10 to 20°C, and after completion of the dropwise addition, the mixture was reacted for 1 hour. The reaction product was poured into 200 ml of water, washed with an aqueous solution of sodium thiosulfate and extracted with methylene chloride. The methylene chloride layer was dried over anhydrous sodium sulfate, and the solvent was evaporated off. The solid thus obtained was washed with n-hexane to obtain 3.5 g of the desired product.

The 2-amino-trifluoromethyl-halogenopyridine per se of this invention has no physiological activity, but can be chemically converted into a substance having high physiological activity. It has been confirmed that the 2-amino-trifluoromethyl-halogenopyridine is a useful intermediate for synthesis of agricultural chemicals and medicines.

For example, 2-amino-3-chloro-5-trifluoromethylpyridine is reacted with 2,6-difluorobenzoyl-isocyanate in toluene at 40°C for 30 minutes, whereby N-(2,6-difluorobenzoyl)-N'-(3-chloro-5-trifluoromethyl-2-pyridyl)urea having a melting point of 214 to 217°C can be obtained. This compound is effective in inhibiting and controlling various pests, particularly noxious insects. For example, in the insecticidal testing of the compound in a concentration of 800 ppm against larvae of diamondback moth in 3rd or 4th instar, a 100% insecticidal effect can be obtained.

**Claims**

1. A 2-amino-trifluoromethyl-halogenopyridine compound characterized by the formula (I):

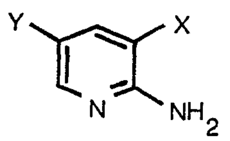

(I)

wherein either X or Y is a trifluoromethyl group and the other is a chlorine atom or a bromine atom.

2. 2-Amino-3-chloro-5-trifluoromethylpyridine.

3. 2-Amino-3-trifluoromethyl-5-chloropyridine.

4. A process for producing a 2-amino-trifluoromethyl-halogenopyridine compound represented by the formula (I):

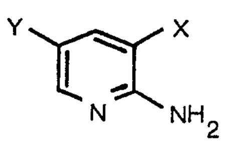

(I)

wherein either X or Y is a trifluoromethyl group and the other is a chlorine atom or a bromine atom, characterized by reacting a 2-halogeno-trifluoromethyl-halogenopyridine represented by the formula (II):

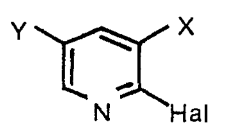

(II)

wherein X and Y are the same as defined hereinbefore, and Hal is a halogen atom, with ammonia at a temperature of 50 to 180°C.

5. A process as claimed in Claim 4, wherein the reaction is carried out at a temperature of 100 to 150°C.

6. A process for producing a 2-amino-trifluoromethyl-halogenopyridine compound represented by the formula (I):

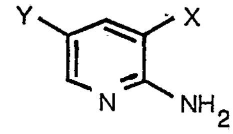

(I)

wherein either X or Y is a trifluoromethyl group and the other is a chlorine atom or a bromine atom, characterized by reacting a 2-halogeno-trifluoromethylpyridine compound represented by the formula (III):

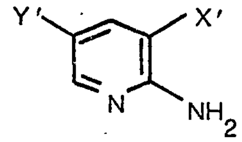

(III)

wherein either X′ or Y′ is a trifluoromethyl group and the other is a hydrogen atom, with a chlorinating or brominating agent.

7. A process as claimed in Claim 6, wherein said halogenating agent is chlorine gas, and the reaction is carried out at a temperature of 0 to 100°C.

8. A process as claimed in Claim 6, wherein said halogenating agent is bromine, and the reaction is carried out at a temperature of 10 to 60°C.

**Patentansprüche**

1. 2-Amino-trifluoromethyl-halogenpyridin-Verbindung entsprechend der Formel (I):

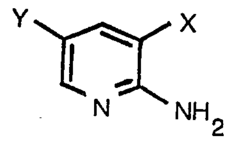

(I)

worin entweder X oder Y eine Trifluoromethylgruppe darstellt und der andere dieser Substituenten ein Chlor- oder ein Bromatom bedeutet.

2. 2-Amino-3-chloro-5-trifluoromethylpyridin.

3. 2-Amino-3-trifluoromethyl-5-chloropyridin.

4. Verfahren zur Herstellung einer 2-Amino-trifluoromethyl-halogenpyridin-Verbindung entsprechend der Formel (I):

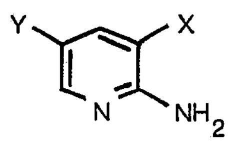

(I)

worin entweder X oder Y eine Trifluormethylgruppe darstellt und der andere dieser Substituenten ein Chlor- oder ein Bromatom bedeutet, gekennzeichnet durch Umsetzung eines 2-Halogeno-trifluoromethyl-halogenopyridins entsprechend der Formel (II):

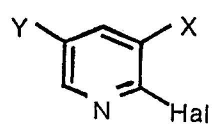

(II)

worin X und Y die vorstehend angegebenen Bedeutungen haben, und Hal ein Halogenatom darstellt, mit Ammoniak bei einer Temperatur von 50 bis 180°C.

5. Verfahren gemäss Anspruch 4, dadurch gekennzeichnet, dass die Umsetzung bei einer Temperatur von 100 bis 150°C durchgeführt wird.

6. Verfahren zur Herstellung einer 2-Amino-trifluoromethyl-halogenpyridin-Verbindung entsprechend der Formel (I):

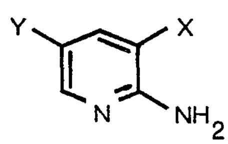

(I)

worin entweder X oder Y eine Trifluoromethylgruppe darstellt und der andere dieser Substituenten ein Chlor- oder ein Bromatom bedeutet, gekennzeichnet durch Umsetzung einer 2-Halogeno-trifluoromethylpyridin-Verbindung entsprechend der Formel (III):

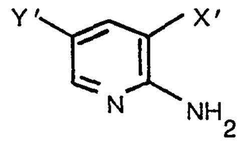

(III)

worin entweder X′ oder Y′ eine Trifluoromethylgruppe darstellt und der andere dieser Substituenten ein Wasserstoffatom bedeutet, mit einem Chlorierungs- oder Bromierungsagens.

7. Verfahren gemäss Anspruch 6, dadurch gekennzeichnet, dass das Halogenierungsagens Chlorgas darstellt und die Reaktion bei einer Temperatur von 0 bis 100°C durchgeführt wird.

8. Verfahren gemäss Anspruch 6, dadurch gekennzeichnet, dass das Halogenierungsagens Brom darstellt und die Reaktion bei einer Temperatur von 10 bis 60°C durchgeführt wird.

**Revendications**

1. Un composé de type amino-2 trifluorométhyl halogénopyridine caractérisé par la formule (I):

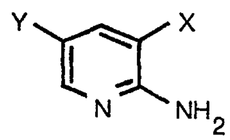

(I)

où X ou Y est un radical trifluorométhyle et l'autre est un atome de chlore ou un atome de brome.

2. Amino-2 chloro-3 trifluorométhyl-5 pyridine.

3. Amino-2 trifluorométhyl-3 chloro-5 pyridine.

4. Un procédé pour produire un composé de type amino-2 trifluorométhyl halogénopyridine représenté par la formule (I)

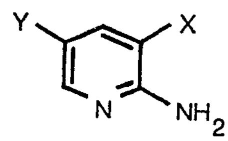

(I)

où X ou Y est un radical trifluorométhyle et l'autre est un atome de chlore ou un atome de brome, caractérisé par la réaction d'une halogéno-2 trifluorométhyl halogénopyridine représentée par la formule (II):

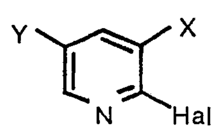

(II)

où X et Y sont comme définis précédemment, et Hal est un atome d'halogène, avec de l'ammoniac à une température de 50 à 180°C.

5. Un procédé comme revendiqué dans la revendication 4 où la réaction est effectuée à une température de 100 à 150°C.

6. Un procédé pour produire un composé de type amino-2 trifluorométhyl halogénopyridine représenté par la formule (I):

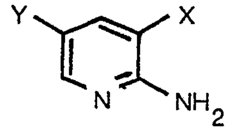

(I)

où X ou Y est un radical trifluorométhyle et l'autre est un atome de chlore ou un atome de brome, caractérisé par la réaction d'un composé de type halogéno-2 trifluorométhyl pyridine représenté par la formule (III):

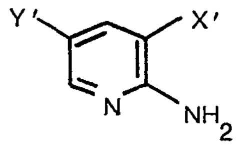

(III)

où X′ ou Y′ est un radical trifluorométhyle et l'autre est un atome d'hydrogène, avec un agent de chloration ou de bromation.

7. Un procédé comme revendiqué dans la revendication 6, où ledit agent d'halogénation est le chlore gazeux et la réaction est effectuée à une température de 0 à 100°C.

8. Un procédé comme revendiqué dans la revendication 6, où ledit agent d'halogénation est le brome et la réaction est effectuée à une température de 10 à 60°C.